(19) Europäisches Patentamt European Patent Office Office européen des brevets

(11) **EP 1 978 394 A1**

(12) **EUROPEAN PATENT APPLICATION**

(43) Date of publication:
08.10.2008 Bulletin 2008/41

(51) Int Cl.:
***G02B 27/00*** (2006.01)

(21) Application number: 07300936.7

(22) Date of filing: 06.04.2007

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Global Bionic Optics Pty Ltd.**
**Sydney South NSW 1235 (AU)**

(72) Inventor: **Mathieu, Gilles Jean**
**34400, LUNEL (FR)**

(74) Representative: **Joly, Jean-Jacques et al**
**Cabinet Beau de Loménie**
**158, rue de l'Université**
**75340 Paris cedex 07 (FR)**

(54) **Optical system for increasing depth of field**

(57) Optical systems are disclosed that are able to increase depth of field. In one aspect, an optical system is disclosed comprising at least one optical clement adapted to provide a spherical aberration in the range of about 0.2 wave to about 5 wave for increasing the depth of field of the optical system.

FIG. 5

**Description**

TECHNICAL FIELD

The present invention relates generally to optical systems for use with imaging systems, including digital image and digital video capture systems amongst others.

BACKGROUND

Typically, in the design of lenses for imaging systems is a technical art, the goal for a wide range of requirements and applications is to minimize aberrations induced by the lens. Aberrations are considered to reduce resolution and contrast. Lens designers seek to avoid aberrations, especially in high quality imaging applications.

Typically, aberrations arise from two origins: optical design and manufacturing errors that should stay in an allowed range of tolerances. Aberrations of a lens can be separated into different kinds, such as Zemike has done, where each aberration has specific effects on an image while commonly adversely affecting resolution and contrast of the image.

A need exists for an optical system that can increase the depth of field in respect of images obtained by such an optical system.

SUMMARY

In accordance with an aspect of the invention, there is provided an optical system, comprising at least one optical element adapted to provide a spherical aberration in the range of about 0.2 wave to about 5 wave for increasing the depth of field of the optical system.

The optical element may comprise at least one lens.

The increased depth of field may be in the light wavelength range of about 300 nanometers to about 1200 nanometers. Alternatively, the increased depth of field may be in the light wavelength range of about 700 nanometers to about 900 nanometers. The optical system may further comprise a system for digitally processing images to increase their Modulation Transfer Function (MTF) at spatial frequencies below the Nyquist Limit.

In accordance with another aspect of the invention, there is provided an optical system for producing an image of an object. The optical system comprises at least one of a lens and an optical element adapted to provide a chromatic aberration in the range of about 0.2 wave to 0.9 wave for increasing the depth of field of the system. The optical system may comprise a plurality of lenses. Further, the optical system may comprise a plurality of optical elements. The increased depth of field may be in the light wavelength range of about 300 nanometers to about 1200 nanometers. The optical system may further comprise a system for digitally processing images to increase their Modulation Transfer Function (MTF) at spatial frequencies below the Nyquist Limit.

The increased depth of field may be in the light wavelength range of about 700 nanometers to about 900 nanometers. Images produced by the optical system may be digitally processed to increase their Modulation Transfer Function at spatial frequencies below the Nyquist Limit.

The spherical aberration may range from about 0.2 wave to about 0.9 wave and provide an increased depth of field and reduced Modulation Transfer Function at spatial frequencies below the Nyquist Limit, the increased depth of field and the reduced Modulation Transfer Function being further increased by digital image processing applied to an image obtained using the optical system.

The spherical aberration may range from about 0.2 wave to about 5 wave providing the increased depth of field, an image produced using the optical system having a reduced Modulation Transfer Function at spatial frequencies below the Nyquist Limit. The optical system may further comprise a module for digital image processing of an image obtained using the system dependent upon a distance determined between an object and the optical system to increase the Modulation Transfer Function at spatial frequencies below the Nyquist Limit. The optical system may further comprise a device for determining the distance between the object and the optical system. Alternatively, a characteristic of an image may used to determine the distance between the object and the optical system. A local Edge Spread Function may be used from a known object in the image to extract the distance between the object and the optical system.

In accordance with still another aspect of the invention, there is provided an optical system, comprising at least one optical element adapted to having an axial chromatic aberration of about 0.4 to about 20 times the Fresnel distance in image space for increasing the depth of field while reducing the magnitude of non-central rings in a Point Spread Function and eliminating undesired zeros in a Modulation Transfer Function.

The increased depth of field may be in the light wavelength range of about 300 nanometers to about 1200 nanometers, or in the light wavelength range of about 700 nanometers to about 900 nanometers.

An image may be obtained using the optical system is digitally processed to increase its Modulation Transfer Function at spatial frequencies below the Nyquist Limit. The digital processing may be dependent upon the distance between an object and the optical system. The optical system may further comprise a device for determining the distance between the object and the optical system. Alternatively, a characteristic of an image may be used to determine the distance between the object and the optical system. A local Edge Spread Function from a known object in the image may be used to extract the object-to-image distance.

BRIEF DESCRIPTON OF THE DRAWINGS

Embodiments of the invention are described hereinafter with reference to the drawings, in which:

Fig. 1 is a chart showing the MTF as a function of spatial frequency;

Fig. 2 is a diagram illustrating ray tracing through a lens or optical element having a spherical aberration in accordance with the embodiments of the invention illustrating increased depth of field (DOF) in which marginal rays and paraxial rays focus at marginal and paraxial focuses, respectively, defining the DOF;

Fig. 3 is a block diagram of an image capture system like that of Fig. 5, with a distance measurement system, device or module 320, but used for capturing images of people's irises, i.e. biometric data;

Fig. 4 is a block diagram of an optical system in accordance with a further embodiment of the invention utilizing a lens with a spherical aberration to provide increased DOF and digital filtering to improve the capture raw image dependent upon distance information obtained derived from the object;

Fig. 5 is a block diagram of an optical system in accordance with another embodiment of the invention utilizing a lens with a spherical aberration to provide increased DOF and digital filtering to improve the capture of a raw image dependent upon distance information obtained from a distance measurement device;

Fig. 6 is a plot of a caustic without spherical aberration;

Fig. 7 is a plot of a caustic with spherical aberration;

Fig. 8 is diagram illustrating a Gauss lens providing a spherical aberration for increased DOF;

Fig. 9A is a block diagram of an optical design without a spherical aberration, and Fig. 9B contains two plots of characteristics of that lens;

Fig. 10A is a block diagram of an optical design with a spherical aberration in accordance with an embodiment of the invention, and Fig. 10B contains two plots of characteristics of that lens;

Fig. 11 is a block diagram of a further optical system using a plano-convex lens and having a spherical aberration to increase DOF;

Fig. 12 is a plot of the raw MTF and an enhanced MTF obtained by digital filtering having a smooth shape to avoid overshoots and artifacts;

Fig. 13 is a plot showing the through-focus MTF with a spherical aberration;

Fig. 14 illustrates the through-focus MTF;

Fig. 15 is a plot of MTF through-focus as a function of normalized defocus; and

Fig. 16 is a plot the Optical Path Difference (OPD) of the spherical aberration where ZF is the Fresnel distance.

DETAILED DESCRIPTIONS

Optical lenses and optical systems for increasing depth of focus (DOF) are described hereinafter. In the following description, numerous specific details, including lens configurations, digital filtering techniques, and the like are set forth. However, from this disclosure, it will be apparent to those skilled in the art that modifications and/or substitutions may be made without departing from the scope and spirit of the invention. In other circumstances, specific details may be omitted so as not to obscure the invention.

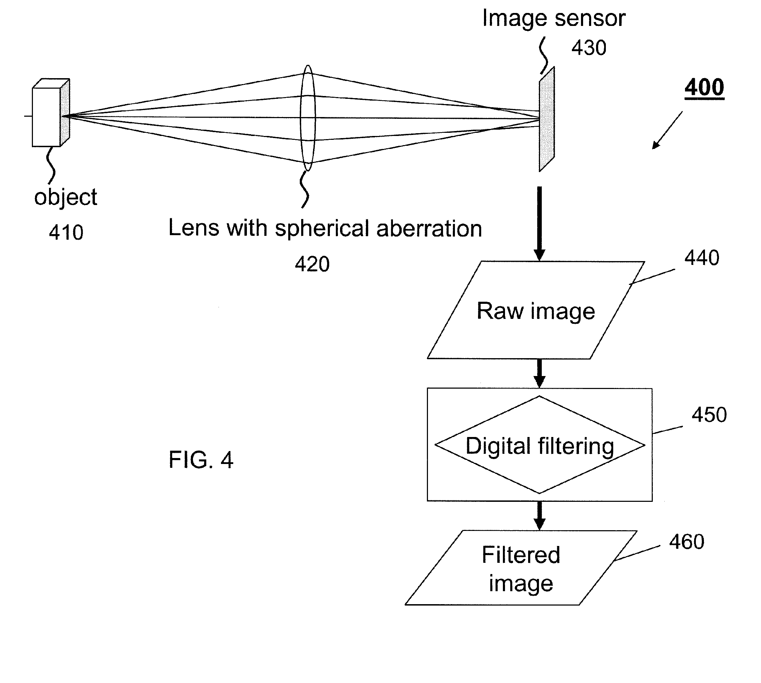

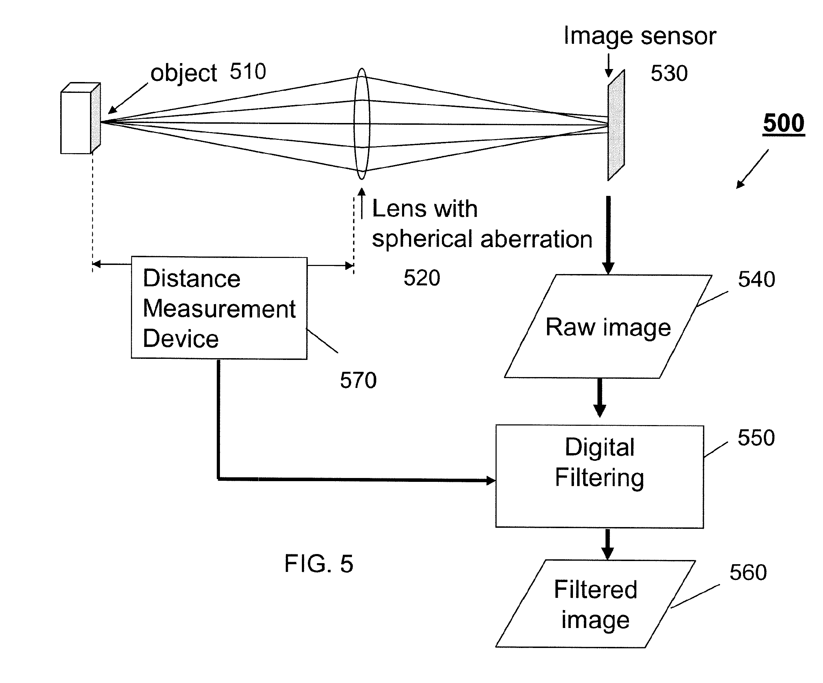

Where reference is made in any one or more of the accompanying drawings to features, which have the same or similar reference numerals, those features have for the purposes of this description the same function(s) or operation(s), unless the contrary intention appears. Like features are generally given like reference numerals in the drawings (e.g. digital filtering 450 and 550 in Figs. 4 and 5, respectively) for the sake of brevity.

In the context of this specification, the word "comprising" has an open-ended, non-exclusive meaning: "including principally, but not necessarily solely", but neither "consisting essentially or" nor "consisting only of". Variations of the word "comprising", such as "comprise" and "comprises", have corresponding meanings.

1. Introduction

Depth of field (DOF) can be defined by rules involving a Point Spread Function (PSF) versus focus. These rules are well known to photographers, cinematographers and more generally in imaging instrumentation. The DOF is related to a specification of resolution loss that is acceptable for a defined application. The circle of confusion diameter is usually the parameter that defines this limit.

Other functions such as the Modulation Transfer Function (MTF) or the Point Spread Function (PSF) are used to characterize image quality in terms of resolution and contrast. These functions are dependent on the focus. The best focus is defined as the image position where the MTF is maximized and the PSF are tightest.

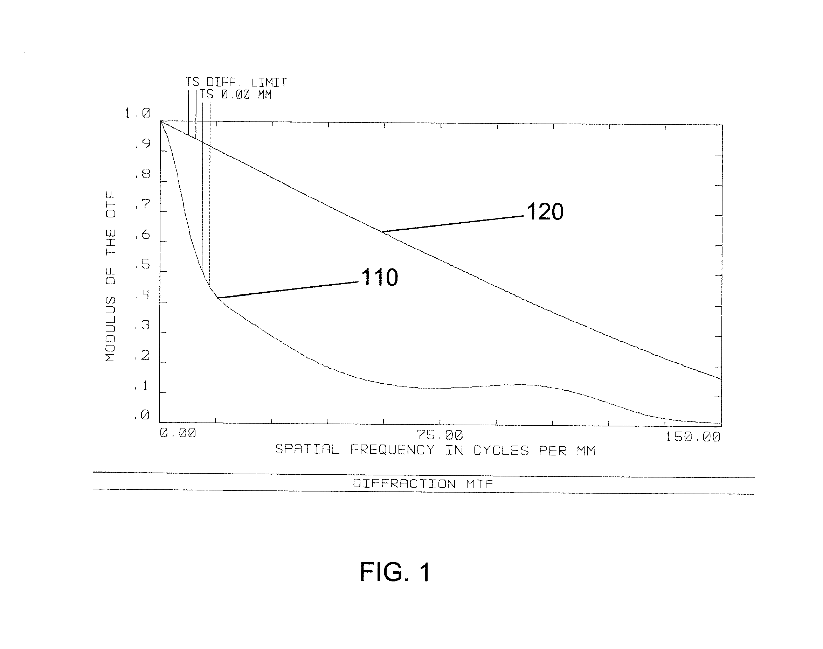

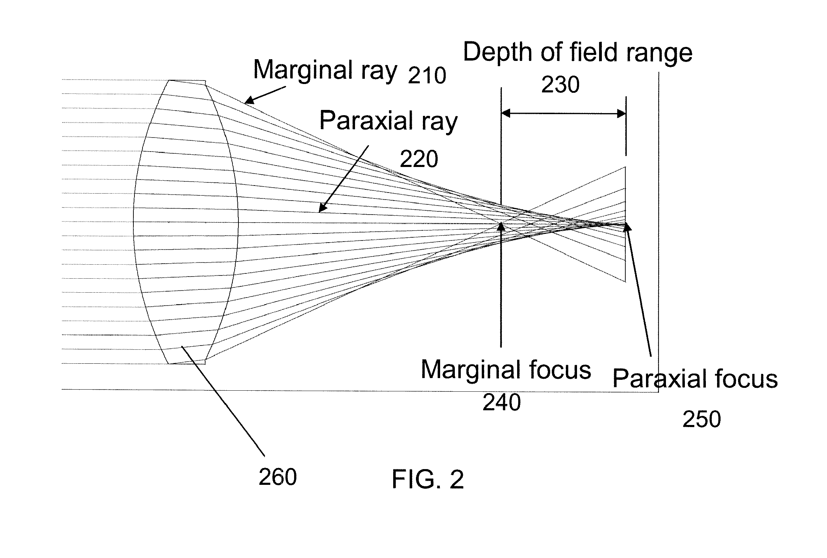

Fig. 1 shows the MTF as a function of spatial frequency. The highest level of MTF at best focus is achieved in the case of no aberrations 120. A spherical aberration reduces the MTF level 110 at best focus too. When an aberration occurs, the best focus position and depth of field are both dependent on the chosen estimating function. The embodiments of the invention increase or enhance the DOF. The methodology of enhancing or increasing DOF involves the use of certain design characteristics of optical lenses and/or other optical elements. In yet another embodiment, digital image processing enhances the quality in case of an image obtained using the noted optical lens and/or optical elements having a specific spherical aberration(s) designed into the optical system. As shown in Fig. 2, the spherical aberration (not depicted) in the lens 260 makes a progressive axial shift of the focus versus the aperture to affect DOF 230, defined by the marginal and paraxial focuses 240 and 250, respectively. The geometrical approach shows different focus positions from the paraxial focus 250 to the marginal focus 230. The paraxial rays 220 focus on the paraxial focus, and the marginal rays focus on the marginal focus 240 in front of the paraxial focus 250 by the DOF 230.

In general, the embodiments of the invention take into account the real diffraction of light. The through-focus characteristic of the image is defined by the evolution of the PSF according diffraction light rules. In one embodiment, the process of optical imaging is coupled with digital image processing to enhance the image quality and sharpness dependent upon the spherical aberration of the optical system. This is described in greater detail hereinafter.

In the following description, aberration amplitude is defined by the wave front error peak to valley that occurs on the exit pupil in wave unit. The term "wave" is a common abbreviation of the term wavelength. In the description hereinafter and the claims, the term "wave" has this meaning.

## 2. An Image Capture System for Any Object

With reference to Fig. 5, an object 510 to be observed can be located at variable distances relative to an image capture system 500 having an image sensor 530 and incorporating or being coupled to an optical system 520 including a lens and/or other optical elements providing the desired spherical aberration to increase DOF. In Fig. 5, only a single lens 520 is depicted for ease of illustration and so as not to obscure the invention. In this embodiment, the image capture system 500 can be operated in conjunction with a distance measurement system 570 that provides the distance information between the optical system 520 and the object 510. As shown in Fig. 5, the lens 520 with the spherical aberration to provide increased DOF focuses the image of the object 510 onto the image sensor 530. The image sensor 530 produces a raw image 540 as its output, which is preferably digital. The raw image 540 and the distance measurement 570 are provided as input for digital image processing, which is preferably digital filtering to produce a filtered image that has increased DOF from the optical system 520 and complementary enhancement of the digital image dependent upon distance information.

The distance information allows more accurate, complementary filtering 550 matching well to the real PSF (Point Spread Function) of the optical system 520, which in turn corresponds to the measured distance. The distance measurement may be synchronized with image capture when the object 510 is in motion to measure the distance accurately to a specified precision corresponding to the acquired image. The distance measurement can be made by any of a number of techniques and devices for measuring distance. One implementation involves using a secondary camera off axis to the lens-and-sensor arrangement 520, 530 to measure the distance. Two or more images can measured from two separate points of view and a triangulation method is then applied.

As shown, the lens 520 having a spherical aberration produces an image of the object 510 on the image sensor 530. Like other aberrations, a spherical aberration reduces the contrast and resolution of the raw image 540. In contrast to conventional lenses that are designed to minimize aberrations to obtain good image quality, the lens 520 of Fig. 5 induces a spherical aberration in the imaging process, which changes the diffracted caustic. The contrast in the raw image 540 deteriorates, but the depth of field increases.

In one implementation, an acquired raw image 540 is digital filtered 550 using software. This digital filtering software 550 processes the raw image 540 according a filtering process calibrated dependent on the distance of the object 510. The parameters of the filter 550 are a function of the distance, and the function is set from a calibration process that involves an MTF measurement at different distances of the object 510 within the increased available DOF. The through-focus MTF can be provided by simulation of the MTF according Huygens-Fresnel's diffraction model or by measurement using a slanted edge, for example. The distance measurement provides a more accurate calibration for the digital filter 550, taking into account the loss of MTF induced by the image sensor 530, any residual defocus offset error, and/or other residual aberrations. The distance measurement is short enough in duration (e.g., about ZF/8 where ZF is the Fresnel distance in the object space) not to under-sample the through-focus MTF and thereby avoid the filtering process producing artifacts. The optical system of Fig. 5 shows the general principle by way of example of an embodiment of the invention in terms of applications general. Another application of the optical system is in iris recognition systems, described hereinafter.

## 3. Another Image Capture System for Any Object

Fig. 4 illustrates an image capture system 400 like that 500 of Fig. 5, but without a distance measurement system, device or module. The object 410, lens 420, image sensor 430, raw image 440, digital filter 450, and filtered image 460 are the same and otherwise have the same configuration as that of the system of Fig. 5, except for omission of the distance measurement device 570. For the sake of brevity, description of like numbered elements (e.g. 4XX and 5XX) is not repeated here. For example, a specific object signature may be practiced instead of measuring the distance between the object 410 and lens 420. Complementary digital image processing can be used to collect the object distance information required by the digital image processing 450, e.g. digital filtering. Optionally, the digital processor 450 uses a local Edge Spread Function from the known object 410 in an image 440 to extract the required distance information.

## 4. A System for Capturing Images of Human Irises

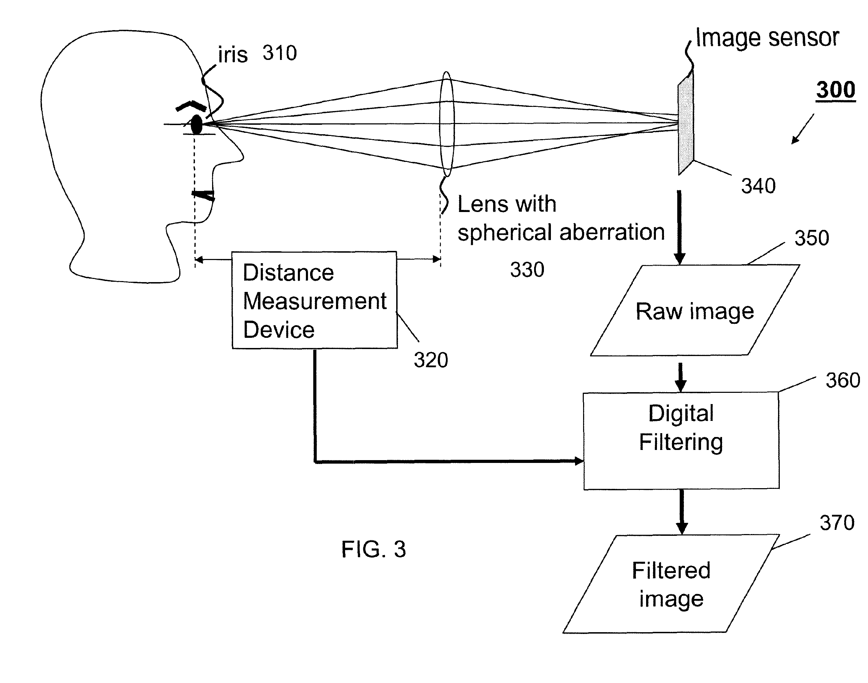

Fig. 3 illustrates an image capture system 300 like that 500 of Fig. 5, with a distance measurement system, device or module 320, but used for capturing images of people's irises, i.e. biometric data. The distance measurement device 320, lens 330, image sensor 340, raw image 350, digital filter 360, and filtered image 370 are the same and otherwise have the same configuration as that of the system of Fig. 5, except that the optical system is used to capture an image of an iris 310, rather than an object 510. Operation of the system 300 corresponds to that of system 500 of Fig. 5 and therefore is not repeated for the sake of brevity. The setting of the digital filter 360 of Fig. 3 can be slightly different from the digital filter 550 of Fig.5 to increase or enhance the depth of field and MTF without ringing, overshoots or artifacts. Generally, other applications in the system 500 may be more tolerant on ringing, artifact, and overshoot. In such a case, the settings of the digital processor provide increased contrast images.

## 5. Lens Characteristics

Typically, an objective lens in an optical system comprises an aligned sequence of one or more individual lenses and/or doublets. In the embodiments of the invention, an objective lens is modified to add a spherical aberration sufficient to produce increased DOF. The lens and/or other optical elements are adapted so that the level of spherical aberration is set according a desired DOF magnification. There are a number of ways to do this once it is appreciated that spherical aberrations can be used to enhance or increase DOF. The lens diopters may be planar, spherical, or aspherical. In an implementation where a number of lenses and/or other optical elements are used, the individual lens component may be conventional, at least in a superficial sense, provided the assembly of components in the optical system provides a desired optical response due to an overall spherical aberration, which is not the goal of conventional designs.

The spherical aberration level is almost the same all over the image field. The field aberration is minimized. The spherical aberration can be achieved in a variety of ways, including:

- A spherical aberration can be added to any optical design as an intrinsic characteristic of that design;
- A parallel plate of optical material having added thickness and a refractive index greater than one (1) in a space where the pupil is far (telecentric);
- Modification of the camber of a lens, where the power is not affected, but the spherical aberration of one lens is directly affected and then the spherical aberration of the whole optical system, i.e. the objective lens, is affected;
- An optical design that adds a spherical-aberration target level in the Merit function used in an optical design software;
- Aspherisation of a lens; and
- Using a lens out of best working points, where a spherical aberration occurs.

In high-resolution optics where the resolution is almost diffraction limited, optical designs avoid aberrations that generate a wave front error (WFE) and reduces the Strehl Ratio (RS), enlarge the Point Spread Function (PSF), and reduce the Modulation Transfer Function (MTF). Conventionally, optical designs for imaging avoid or minimize such aberrations. In contrast, the embodiments of the invention comprise adjusting, sometimes slightly, the design of an optical system to have mainly a spherical aberration. A low chromatic aberration can enhance the results in several cases.

The designed spherical aberration reduces the contrast of an image obtained using the optical system. The level of the MTF 110 is reduced from the frequency at 0 Hz to the cutoff frequency, relative to curve 120, as shown in Fig. 1. The cutoff frequency is not significantly reduced, and consequently nearly the entire spatial spectrum is available. Information remains in the image, but with a lower contrast. The contrast can be restored by digital filtering in accordance with some embodiments of the invention.

The spherical aberration increases the DOF in the sense that the high spatial frequencies stay available for a longer range of defocus with spherical aberration. The digital filtering used can restore the contrast and produce a good quality image using the increased or enhanced DOF.

The relevant characteristics of the spherical aberration are:

- The revolute symmetry of wave front error. The phase of each compound is null and the Optical Transfer Function (OTF) is symmetrical revolute.

The one dimensional MTF measurement on a slanted edge provides the required information for digital filtering to restore the two-dimensional image.

- One of the two directions, rear or forward, defocus moving from the best focus does not have a zero in the MTF and therefore no contrast inversion according to the sign (positive or negative) of the spherical aberration. The image can be restored on this side of defocus without ringing, overshoot, and artifacts.

- A limited spherical aberration at $0.75 \times \lambda$ significantly increases DOF without zero in the MTF on one defocus side. Beyond $0.75 \times \lambda$, the zero occurs on both sides of defocus from the best focus.

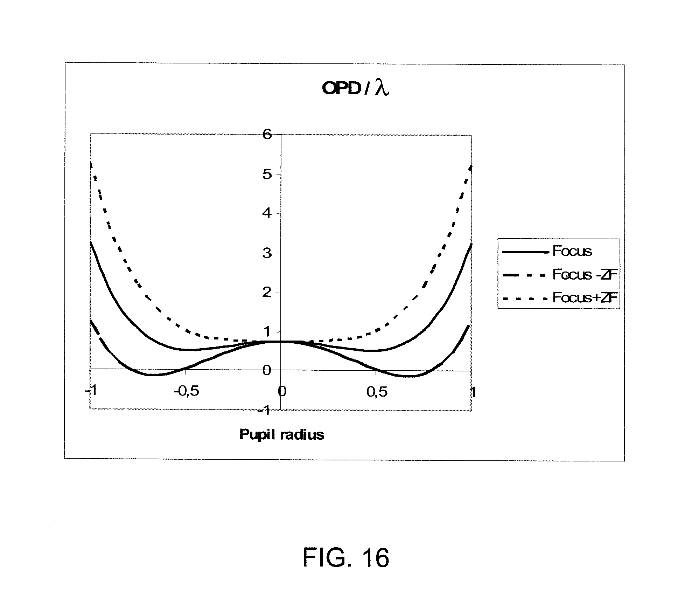

Fig. 16 illustrates the Optical Path Difference (OPD) of the spherical aberration involves the phase of a wave that is $2\pi$ OPD. In focus, the pupil area where phase variation is within +/- $\pi$/2 doesn't cover the whole pupil area but remains close. The characteristic of this OPD is to reduce decreasing of the pupil area, where the phase stays within +/- $\pi$/2. Such a phase distribution or wave front does not allow a zero on the Strehl Ratio within a defocus range of several times the Fresnel distance and does not allow a zero in the MTF. While there is a loss of contrast on the MTF and the loss of Strehl ratio at the best focus, the information in the image is not erased by any zero or contrast inversion.

Use of a symmetrical revolute wave front, which has progressive variation of curvature as the spherical aberration, extends the depth of field with or without digital processing to amplify and restore reduced contrast.

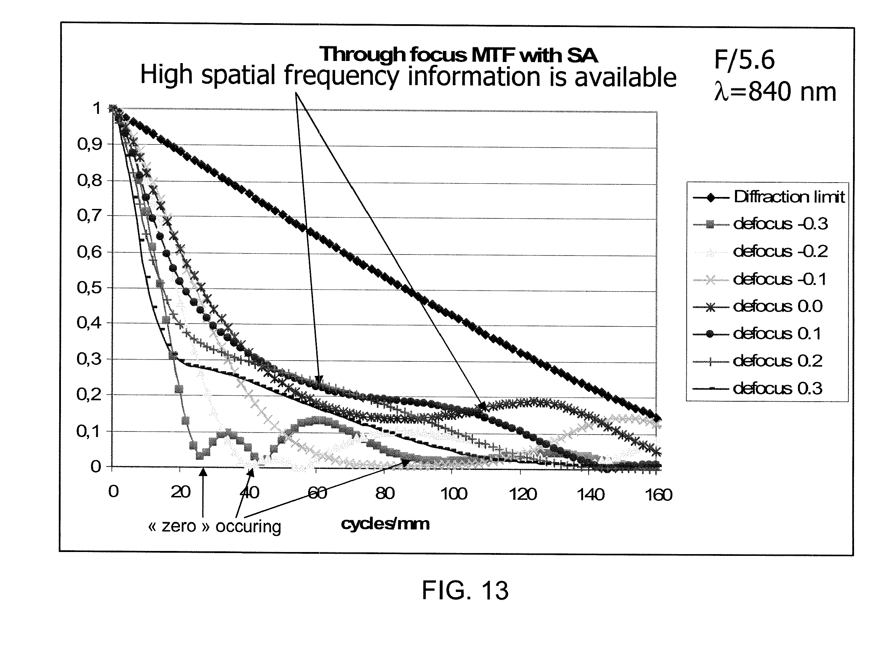

Fig. 13 is a plot showing the through-focus MTF with a spherical aberration, including curves for the diffraction limit and several defocus values. As can be seen from the plots, high spatial frequency information is available (for F/5.6 and $\lambda$=840 nm).

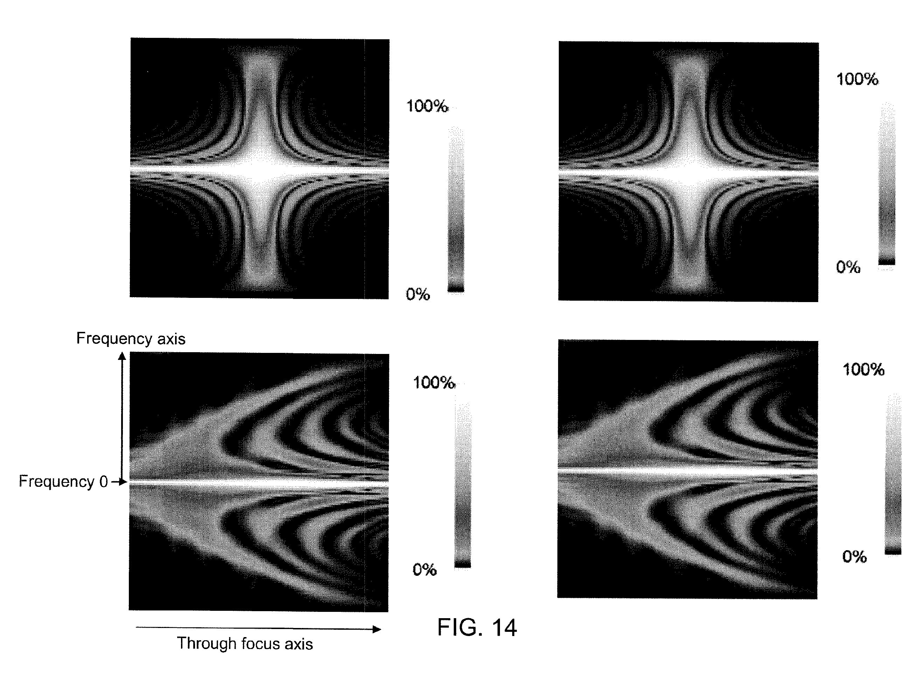

The right side of Fig. 14 shows the through-focus MTF. The side of defocus has no "zero" in the MTF, and the DOF available increases to restore a good image. In contrast, for a conventional system, the MTF decreases quickly in medium and high frequencies with the defocus. This is depicted in the upper two images of Fig. 14. The apparition of a "zero" beyond the depth of field limit erases all information required to maintain the main information of the image. The "zero" occurring is shifted farther into defocus in case of spherical aberration

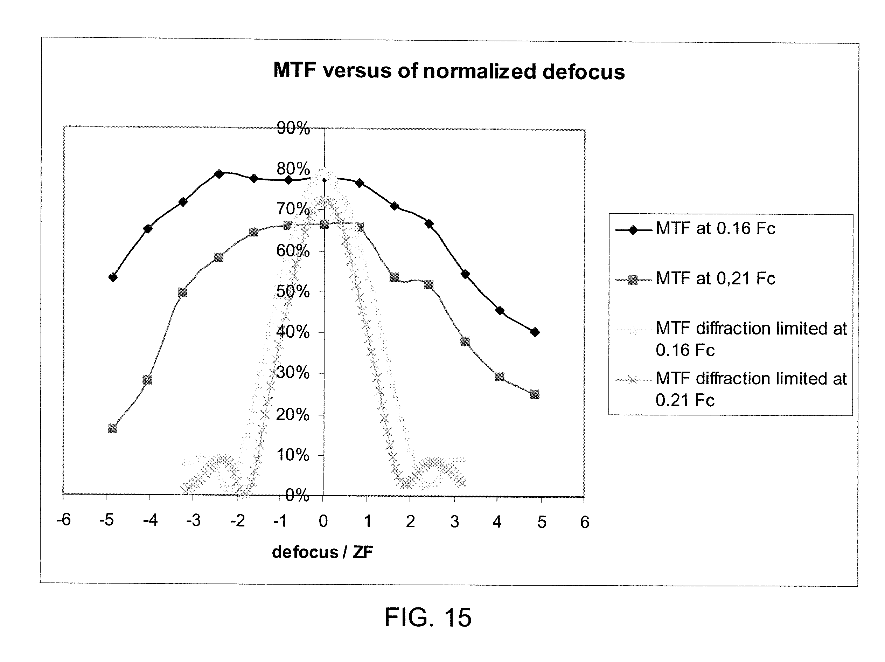

Fig. 15 is a plot of MTF through focus as a function of normalized defocus, in which a comparison is made between the measured MTF on filtered images (0.16 Fc and 0.21 Fc) and the diffraction limited MTF (at 0.16 Fc and 0.21 Fc). In the plot, Fc is the cut off spatial frequency on best focus diffraction limited MTF. Further, ZF is the Fresnel distance:

$$ZF = \frac{\lambda}{NA^2},$$

where NA is the numerical aperture and $\lambda$ is the wavelength.

## 5. Imaging Processing

The imaging processing is implemented as a digital filter optimized to restore the MTF as a smooth function that decreases continuously to avoid overshoots, ringing and artifacts in the image.

Noise amplification is frequently a problem in any sharpening filtering process. An optimized gain function that takes in account the power spectrum of noise similar to a Wiener's filter is applied to reduce the nose amplification. The gain function applied on the MTF depends of the object distance (see Fig. 5). The MTF as a function of distance is acquired by a calibration process. The MTF is measured in the expected depth of field range by sampling step defocus distance less than 1/8 Fresnel's distance to avoid any under sampling loss of information. At each step of defocus distance, digital filtering is applied to restore the MTF on the processed image dependent upon the measured MTF.

The use of this filter requires knowledge of the defocus distance. The defocus distance can be measured by any of a number of devices and techniques for measuring the distance of an object. The filter parameter at a distance between two separate neighbor steps of focus distance can be calculated by the linear interpolation of the parameters of these two steps to reduce the error distance effect.

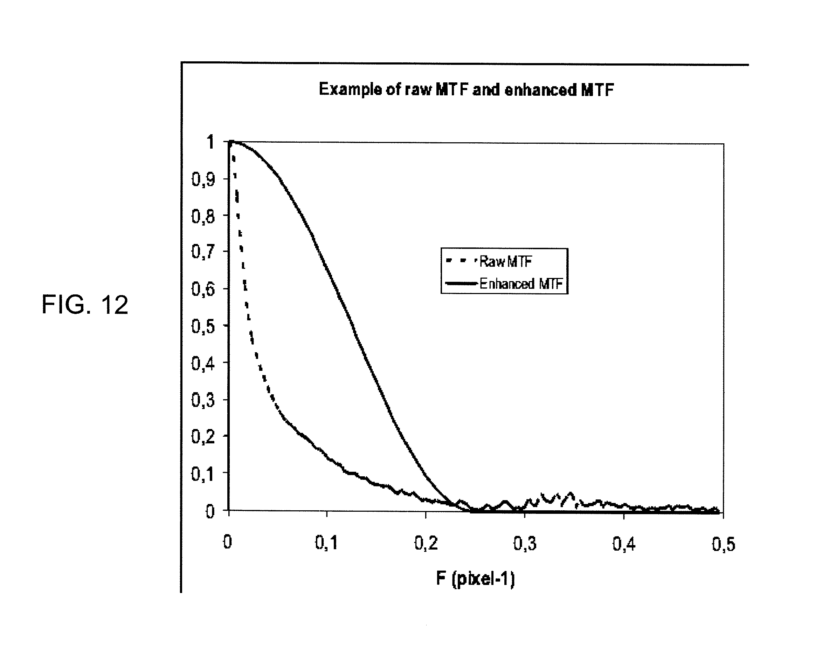

Fig. 12 shows the raw MTF and the enhanced MTF having a smooth shape to avoid overshoots and artifacts. The applied gain of the digital filter is optimized or enhanced to obtain the maximum MTF while controlling the gain on noise.

Unlike conventional lenses, lenses and other optical elements in accordance with the embodiments of the invention exploit spherical aberrations. Any lens design can be changed, in some cases even slightly, to get the required spherical aberration. The optical effect on DOF is effected by the spherical aberration. In the light of this disclosure, in theory, increasing depth of field can be independent of the lens design principle. A different optimization can be applied on any of a number of lens designs to produce the spherical aberration and obtain increased depth of field. Thus, increased depth of field arising from spherical aberrations extends well beyond the examples described herein and shown in the drawings, by way of example. Set out below are several examples involving this concept.

## 6. A First Example

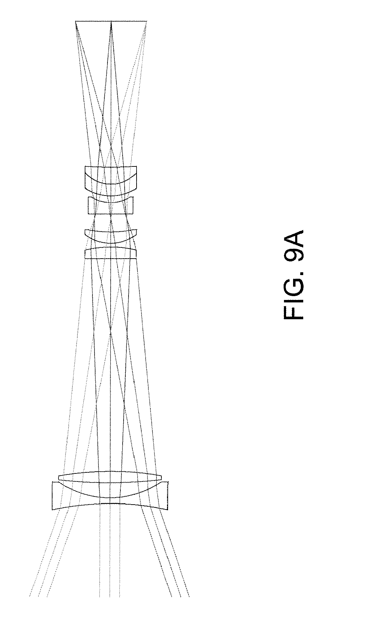

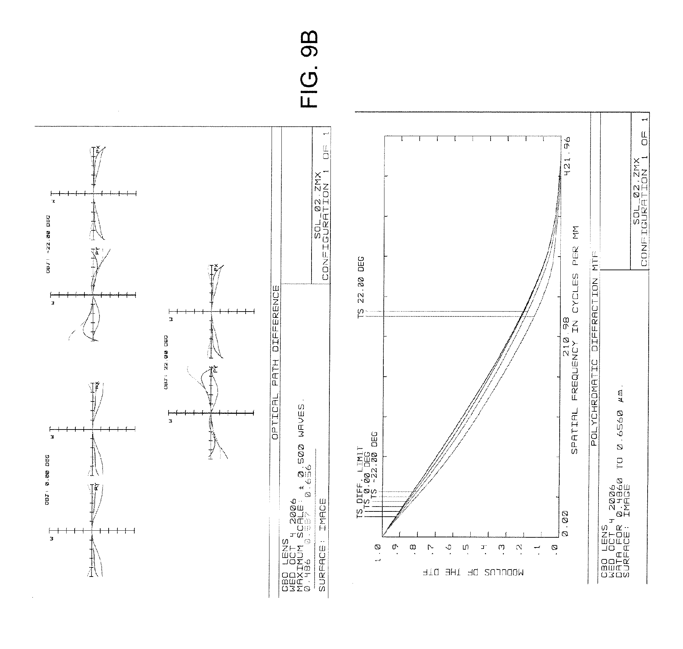

Fig. 9A is an example of an optical design without a spherical aberration, and Fig. 9B contains two plots of characteristics of that lens with a spherical aberration. The optical path difference plot of Fig. 9B shows some residual aberrations, mainly secondary chromatic aberrations and aberrations of coma in the field. The aberrations are low compared to the wavelength as demonstrated in the MTF plot of Fig. 9B, which is close to the diffraction limit MTF.

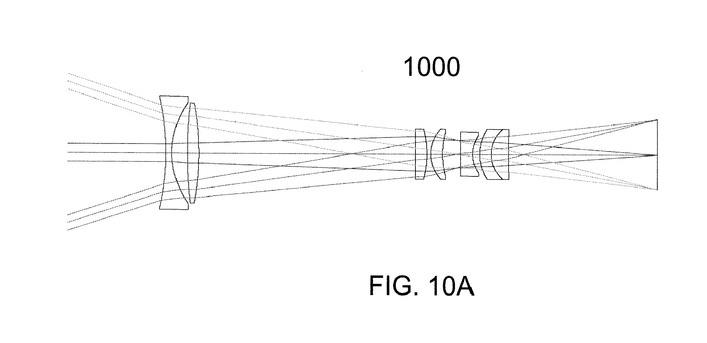

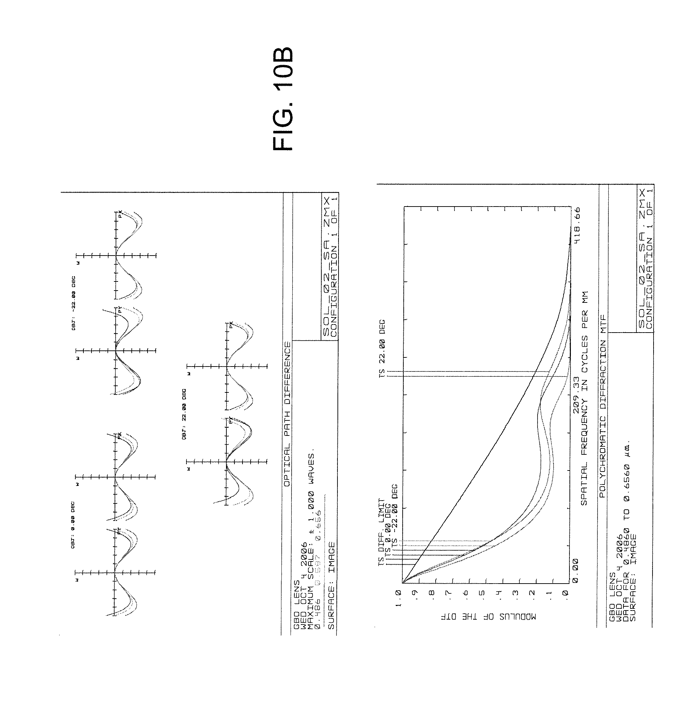

Fig. 10A is an example of the optical design like that of Fig. 9A but with the spherical aberration, arising from changes to the lens elements 1000, in accordance with an embodiment of the invention to produce the effect of increased depth of field.

The increased DOF arising from a spherical aberration is produced by changing slightly the radius of curvature of the two lenses 1000.

## 7. A Second Example

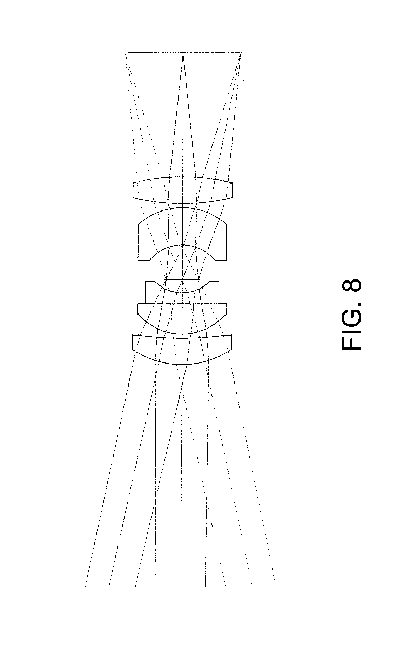

Fig. 8 illustrates a Gauss lens having a spherical aberration in accordance with a further embodiment of the invention. This example illustrates that a "conventional" optical design can be modified to have a spherical aberration that increases the depth of field for the system.

## 8. A Further Example

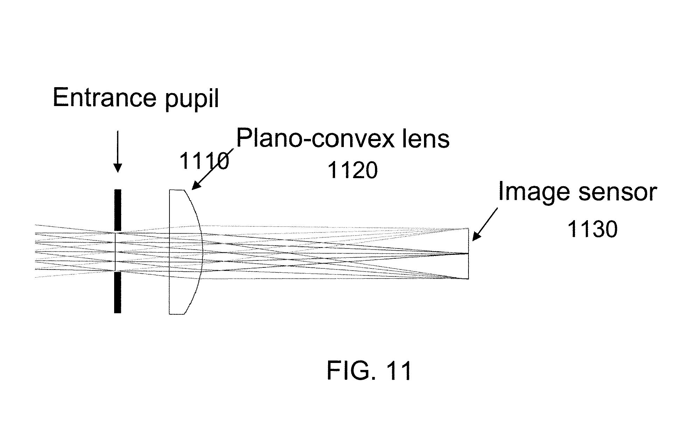

Fig. 11 illustrates a further optical system having a spherical aberration to increase or enhance depth of field. Light is input via an entrance pupil 1110 and passes through a plano-convex lens 1120 with the spherical aberration. The light focuses on an image sensor 1130. This simple design provides the required spherical aberration at F/6.6. Such a design, for example, matches well to working conditions for an iris recognition system or application. The light that is reflected from the eye pupil and collected through an entrance pupil or diaphragm 1110 (diameter of 8 mm located 11 mm) located in front of the plano-convex fused-silica lens 1120. However, the lens 1120 may be made from any glass or molted plastic. The location of the pupil 1110 is chosen at the place where coma aberration does not occur in the field at 11 mm ahead of the plane side of the lens 1110. Astigmatism is insignificant. The main aberrations are:

- A spherical aberration. The mounting side is opposite of the minimum of the spherical aberration. The level of aberration with fused silica lens is 0.75 wave in near infrared at F/6.6 at a focal length of 50mm.
- A chromatic aberration. The lens 1120 in this example is not corrected for chromatic aberration, which can be advantageous to increase the DOF when used with a broad spectrum lighting by erasing any zeroes in the MTF. Otherwise, the chromatic aberration is limited with an Abbe number > 60 to not damage the MTF by lateral color in the edge of field.

- Curvature of field. The curvature of field has to be taken in account to set the best focus plane. Its effect is reduced by the gain on the depth of field.

This design is well suited for an iris recognition application. The design matches well to many other applications that requires similar optical characteristics. Preferably, a working aperture within the range [F/8 to F/4] can be used. Advantageously, this system is simple and inexpensive. Digital filtering can restore image quality and sharpness.

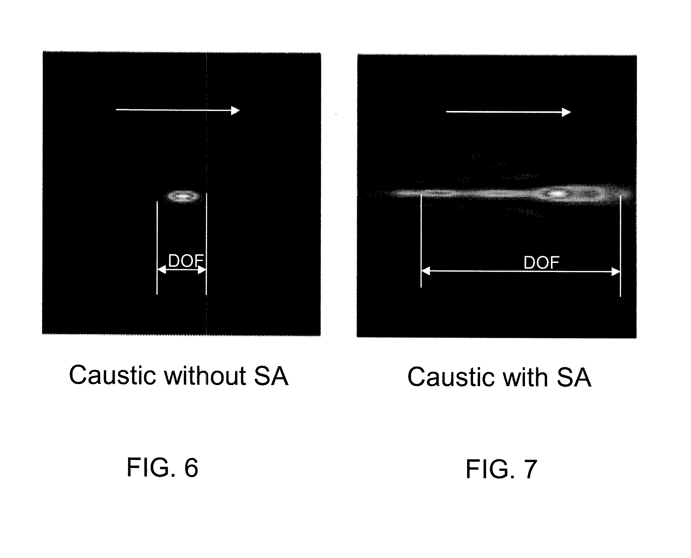

Fig. 6 shows a simulated diffraction caustic without a spherical aberration (SA) and the associated DOF, and Fig. 7 shows a caustic with an SA with increased DOF. Figs. 6 and 7 show the DOF enhancement with the spherical aberration.

Optical lenses and optical systems for increasing depth of focus have been described. While only a small number of embodiments of the invention have been described, in view of this disclosure, it will be apparent to one skilled in the art that modifications and/or substitutions can be made without departing from the scope and spirit of the invention.

## Claims

1. An optical system, comprising:

   at least one optical element adapted to provide a spherical aberration in the range of about 0.2 wave to about 5 wave for increasing the depth of field of said optical system.

2. The optical system according to claim 1, wherein said optical element comprises at least one lens.

3. The optical system according to claim 1, wherein said increased depth of field is in the light wavelength range of about 300 nanometers to about 1200 nanometers.

4. The optical system according to claim 1, wherein said depth of field is in the light wavelength range of about 700 nanometers to about 900 nanometers.

5. The optical system according to claim 3 or 4, further comprising a system for digitally processing images to increase their Modulation Transfer Function (MTF) at spatial frequencies below the Nyquist Limit.

6. An optical system for producing an image of an object, said optical system comprising:

   at least one of a lens and an optical element adapted to provide a chromatic aberration in the range of about 0.2 wave to 0.9 wave for increasing the depth of field of said system.

7. The optical system according to claim 6, comprising a plurality of lenses.

8. The optical system according to claim 6 or 7, comprising a plurality of optical elements.

9. The optical system according to any one of claims 6 to 8, wherein said increased depth of field is in the light wavelength range of about 300 nanometers to about 1200 nanometers.

10. The optical system according to claim 9, further comprising a system for digitally processing images to increase their Modulation Transfer Function (MTF) at spatial frequencies below the Nyquist Limit.

11. The optical system according to claim 6, wherein said increased depth of field is in the light wavelength range of about 700 nanometers to about 900 nanometers.

12. The optical system according to claim 11, wherein images produced by said optical system are digitally processed to increase their Modulation Transfer Function at spatial frequencies below the Nyquist Limit.

13. The optical system according to claim 1, wherein said spherical aberration ranges from about 0.2 wave to about 0.9 wave and provides an increased depth of field and reduced Modulation Transfer Function at spatial frequencies below the Nyquist Limit, said increased depth of field and said reduced Modulation Transfer Function being further increased by digital image processing applied to an image obtained using said optical system.

14. The optical system according to claim 1, wherein said spherical aberration range from about 0.2 wave to about 5 wave providing said increased depth of field, an image produced using said optical system having a reduced Modulation Transfer Function at spatial frequencies below the Nyquist Limit.

15. The optical system according to claim 14, further comprising means for digital image processing of an image obtained using said system dependent upon a distance determined between an object and said optical system to increase the Modulation Transfer Function at spatial frequencies below the Nyquist Limit.

16. The optical system according to claim 14, further comprising a device for determining the distance between said object and said optical system.

17. The optical system according to claim 14, wherein a characteristic of an image is used to determine the distance between said object and said optical system.

18. The optical system according to claim 17, wherein a local Edge Spread Function is used from a known object in said image to extract the distance between said object and said optical system.

19. An optical system, comprising:

at least one optical element adapted to having an axial chromatic aberration of about 0.4 to about 20 times the Fresnel distance in image space for increasing the depth of field while reducing the magnitude of non-central rings in a Point Spread Function and eliminating undesired zeros in a Modulation Transfer Function.

20. The optical system according to claim 19, wherein said increased depth of field is in the light wavelength range of about 300 nanometers to about 1200 nanometers.

21. The optical system according to claim 19, wherein said increased depth of field is in the light wavelength range of about 700 nanometers to about 900 nanometers.

22. The optical system according to any one of claims 19 to 21, wherein an image obtained using said optical system is digitally processed to increase its Modulation Transfer Function at spatial frequencies below the Nyquist Limit.

23. The optical system according to claim 22, wherein said digital processing is dependent upon the distance between an object and said optical system.

24. The optical system according to claim 23, further comprising a device for determining the distance between said object and said optical system.

25. The optical system according to claim 23, wherein a characteristic of an image is used to determine the distance between said object and said optical system.

26. The optical system according to claim 25, wherein a local Edge Spread Function from a known object in the image is used to extract the object-to-image distance.

TS DIFF. LIMIT
TS 0.00 MM

MODULUS OF THE OTF

1.0
.9
.8
.7
.6
.5
.4
.3
.2
.1
.0

120

110

0.00                    75.00                    150.00
SPATIAL FREQUENCY IN CYCLES PER MM

DIFFRACTION MTF

FIG. 1

Marginal ray 210

Depth of field range
230

Paraxial ray
220

Marginal focus
240

Paraxial focus
250

260

FIG. 2

FIG. 3

Image sensor
430

400

object
410

Lens with spherical aberration
420

440
Raw image

450
Digital filtering

460
Filtered image

FIG. 4

FIG. 5

EP 1 978 394 A1

Caustic without SA

FIG. 6

Caustic with SA

FIG. 7

EP 1 978 394 A1

FIG. 8

FIG. 9A

OPTICAL PATH DIFFERENCE

CBO LENS
WED OCT 4 2006
MAXIMUM SCALE: ± 0.500 WAVES.
0.486    0.587    0.656

SURFACE: IMAGE

SOL_02.ZMX
CONFIGURATION 1 OF 1

FIG. 9B

POLYCHROMATIC DIFFRACTION MTF

CBO LENS
WED OCT 4 2006
DATA FOR 0.4860 TO 0.6560 μm.
SURFACE: IMAGE

SOL_02.ZMX
CONFIGURATION 1 OF 1

1000

FIG. 10A

## FIG. 10B

Entrance pupil

Plano-convex lens
1120

1110

Image sensor
1130

FIG. 11

EP 1 978 394 A1

FIG. 12

Example of raw MTF and enhanced MTF

Legend:
- - - Raw MTF
—— Enhanced MTF

F (pixel-1)

EP 1 978 394 A1

FIG. 13

FIG. 14

Frequency axis

Frequency 0

Through focus axis

100%

0%

EP 1 978 394 A1

**MTF versus of normalized defocus**

Legend:
- MTF at 0.16 Fc
- MTF at 0,21 Fc
- MTF diffraction limited at 0.16 Fc
- MTF diffraction limited at 0.21 Fc

x-axis: defocus / ZF

**FIG. 15**

FIG. 16

EP 1 978 394 A1

**European Patent Office**

## EUROPEAN SEARCH REPORT

Application Number

EP 07 30 0936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/050409 A1 (GEORGE NICHOLAS [US] ET AL) 9 March 2006 (2006-03-09)<br>* abstract *<br>* paragraph [0010] - paragraph [0012] *<br>* paragraph [0051] - paragraph [0056] *<br>* paragraph [0066] *<br>* paragraph [0071] - paragraph [0072] *<br>----- | 1-5, 13-18 | INV.<br>G02B27/00 |
| X | WO 2006/083488 A (PSC SCANNING INC [US]) 10 August 2006 (2006-08-10)<br>* abstract *<br>* paragraph [0009] - paragraph [0015] *<br>* paragraph [0020] - paragraph [0022] *<br>* figure 2 *<br>----- | 6-12, 19-26 | |

TECHNICAL FIELDS SEARCHED (IPC)

G02B

The present search report has been drawn up for all claims

| Place of search | Date of completion of the search | Examiner |
|---|---|---|
| Munich | 31 August 2007 | Schenke, Cordt |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 30 0936

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2007

| Patent document cited in search report | | Publication date | Patent family member(s) | | Publication date |
|---|---|---|---|---|---|
| US 2006050409 | A1 | 09-03-2006 | AU 2005283143 A1 | | 16-03-2006 |
| | | | CA 2577735 A1 | | 16-03-2006 |
| | | | EP 1789830 A2 | | 30-05-2007 |
| | | | KR 20070057231 A | | 04-06-2007 |
| | | | WO 2006028527 A2 | | 16-03-2006 |
| WO 2006083488 | A | 10-08-2006 | US 2006171041 A1 | | 03-08-2006 |

EPO FORM P0459

For more details about this annex : see Official Journal of the European Patent Office, No. 12/82